(19) Europäisches Patentamt
European Patent Office
Office européen des brevets

(11) Veröffentlichungsnummer: **0 263 956 B1**

(12) **EUROPÄISCHE PATENTSCHRIFT**

(45) Veröffentlichungstag der Patentschrift: **16.10.91**

(51) Int. Cl.$^5$: **C07F 15/00**, C07C 247/00, C07C 213/00, C07H 15/26, C07C 237/00

(21) Anmeldenummer: **87112768.4**

(22) Anmeldetag: **02.09.87**

Die Akte enthält technische Angaben, die nach dem Eingang der Anmeldung eingereicht wurden und die nicht in dieser Patentschrift enthalten sind.

# (54) Cis-Platin-Komplexe, Verfahren zu ihrer Herstellung und diese Verbindungen enthaltende pharmazeutische Mittel.

(30) Priorität: **08.09.86 DE 3630497**

(43) Veröffentlichungstag der Anmeldung:
**20.04.88 Patentblatt 88/16**

(45) Bekanntmachung des Hinweises auf die Patenterteilung:
**16.10.91 Patentblatt 91/42**

(84) Benannte Vertragsstaaten:
**AT BE CH DE ES FR GB GR IT LI LU NL SE**

(56) Entgegenhaltungen:
**EP-A- 0 098 135**
**EP-A- 0 174 542**
**EP-A- 0 231 847**

**Schröder, Rufer, Schmiechen "Arzneimittelchemie I, George Thieme Verlag, Stuttgart, 1976, Seiten 24-39.**

(73) Patentinhaber: **BEHRINGWERKE Aktiengesellschaft**
**Postfach 1140**
**W-3550 Marburg 1(DE)**

(72) Erfinder: **Kolar, Cenek, Dr.**
**Deutschhausstrasse 20**
**W-3550 Marburg(DE)**
Erfinder: **Dehmel, Konrad**
**Blumengarten 11**
**W-3550 Marburg(DE)**
Erfinder: **Kraemer, Hans Peter, Dr.**
**Birkenweg 16**
**W-3550 Marburg(DE)**

(74) Vertreter: **Klein, Otto, Dr. et al**
**Hoechst AG Zentrale Patentabteilung Postfach 80 03 20**
**W-6230 Frankfurt am Main 80(DE)**

**EP 0 263 956 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft neue Cis-Platin-Komplex-Verbindungen mit einem Propan-1,3-diamin-Derivat als Liganden, ein Verfahren zu ihrer Herstellung und ein pharmazeutisches Mittel, in dem diese neuen Verbindungen enthalten sind.

Cis-Platin-Komplexe der allgemeinen Formel Cis-$L_2$-$PtX_2$,wobei L ein neutraler Ligand wie $NH_3$ oder ein organisches Amin und X ein anionischer Ligand wie Chlorid oder ein Anion einer organischen Säure ist, besitzen antitumorale Wirksamkeit (Cisplatin; Current Status and New Developments Eds. A. W. Prestayko, S. T. Crooke, S. K. Carter, Academic Press, 1980, 149-191).

Cis-Platin-(II))-diamin-dichlorid ist als Arzneimittel eingeführt.

In EPA 0 098 135 (A2) sind Cis-Platin-Komplexe beschrieben, die als Liganden Alkyl- oder Hydroxyalkylsubstituierte Propan-1,3-diamino-Derivate besitzen. In DE 33 37 333 (A1) und GB 20 24 823 (A) sind ebenfalls Alkyl-, Aryl- oder Arylalkylpropan-1,3-diamin-Platin-Komplexe beschrieben.

In der DE 34 32 320 (A1) sind symmetrische Propan-1,3-diamin-Platin-Komplexe beschrieben, die am C-2 zwei Alkyloxymethylsubstituenten tragen.

Die Nieren- und Knochenmarktoxizität der Alkyldiaminoplatin-Komplexe sowie ihre geringe Löslichkeit sind von Nachteil.

Überraschenderweise zeigte sich, daß die synthetischen, bisher unzugänglichen Verbindungen 2-Ethyl-2-methoxymethyl-propan-1,3-diamin-N,N-platin-(II)-dichlorid und das entsprechende Malonat-Derivat hohe zytostatische Wirksamkeit im "in vivo"-Test mit der L1210 Tumorzellinie aufweisen, und daß diese Verbindungen in vitro nur partiell kreuzresistent zu dem in der Klinik aufgeführten Arzneimittel Cis-Platin-(II)-diamin-dichlorid sind.

Ausgehend von dieser Erkenntnis hatte es sich die vorliegende Erfindung zur Aufgabe gestellt, neue am C-2-Atom asymmetrisch substituierte Propan-1,3-diamino-platin-Komplexe herzustellen, und deren pharmakologische Nützlichkeit als Zytostatikum zu überprüfen.

Gelöst wurde diese Aufgabe durch die Verbindungen der Formel I, die in der Prüfung auf zytostatische Wirksamkeit auffielen.

Gegenstand der Erfindung sind Cis-Platin-(II)-diamin-Komplexe der Formel I

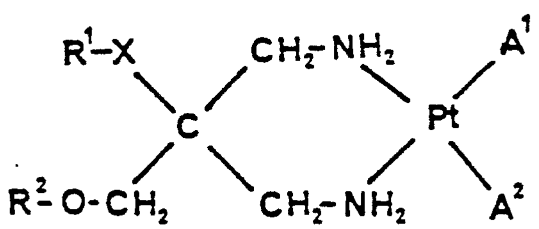

I

worin

| | |
|---|---|
| $R^1$ | ein Wasserstoffatom oder eine Alkylgruppe der Formel $CH_3(CH_2)_n$- mit n = 0 bis 5, |
| $R^2$ | ein Wasserstoffatom, wenn X eine Carbamoylgruppe ist, eine Alkylgruppe mit 1 bis 6 Kohlenstoffatomen, eine etherartig gebundene Gruppe der Formel $R^3$-O-$CH_2$-$(CHR^4)_m$-$CH_2$-, in der |
| $R^3$ | ein Wasserstoffatom oder eine Alkylgruppe mit 1 bis 6 Kohlenstoffatomen, |
| $R^4$ | eine Hydroxygruppe oder eine Alkyloxygruppe mit 1 bis 3 Kohlenstoffatomen und m = 0 bis 2 sind, und $R^2$ weiter eine etherartig gebundene Gruppe der Formel H-$(CH_2)_a$-$(O$-$(CH_2)_b)_c$- mit a = O bis 4, b = 1 bis 4 und c = 1 bis 7 oder einen O-glycosidisch gebundenen Rest der Formel |

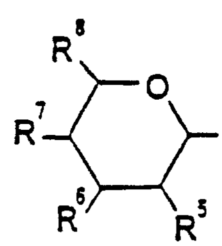

in dem

$R^5$, $R^6$ und $R^7$ unabhängig voneinander ein Wasserstoffatom oder eine Hydroxygruppe und

$R^8$ ein Wasserstoffatom, eine Methyl- oder Hydroxymethylgruppe sind,

X eine Methylen- oder eine Carbamoylgruppe oder eine kovalente Bindung zwischen $R^1$ und dem C-2-Atom,

$A^1$ und $A^2$ gleich sind und die Hydroxygruppe, Chlorid, Bromid, Jodid, Nitrat, Acetat oder Trifluoracetat oder

$A^1$ Sulfat oder Carbonat und $A^2$ $H_2O$ oder

$A^1$ und $A^2$ zusammen das Dianion einer organischen Säure wie Oxal-, Malon-, Hydroxymalon-, Ethylmalon-, 1,1-oder 1,2-Cyclobutan-dicarbon-, Phthal-, 3- oder 4-Carboxyphthal-, 3,4-Dicarboxyphthal oder N-(Carbamoylmethyl)-iminodiessigsäure darstellen.

Bevorzugt werden im Rahmen der Erfindung Verbindungen der allgemeinen Formel I, worin die Reste

$R^1$-X eine Methyl- oder Ethylgruppe,

$R^2$ eine Methyl- oder Ethylgruppe, eine etherartig gebundene Gruppe der Formel

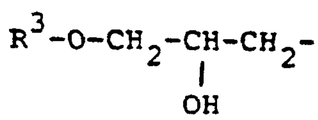

oder $R^3$-$(OCH_2CH_2)_c$-

mit

$R^3$ ein Wasserstoffatom oder ein Methylrest und c = 1,2,3 oder 6 oder ein O-glycosidisch gebundener Rest der Formel

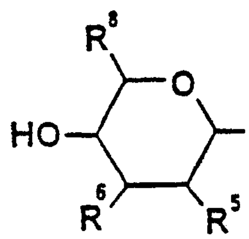

wobei

$R^5$ und $R^6$ unabhängig voneinander ein Wasserstoffatom oder eine Hydroxygruppe,

$R^8$ ein Wasserstoffatom, eine Methyl- oder Hydroxymethylgruppe,

$A^1$ und $A^2$ gleich sind und die Hydroxygruppe, Chlorid oder Nitrat oder

$A^1$ und $A^2$ zusammen das Dianion der Malon-, 1,1-Cyclobutandicarbon- oder N-(Carbamoylmethyl)-iminodiessigsäure sind

sowie Verbindungen der allgemeinen Formel I, worin die Reste

$R^1$-X eine Acetamidogruppe und

$R^2$ ein Wasserstoffatom sind und

$A^1$ und $A^2$ die zuletzt genannte Bedeutung besitzen.

Die erfindungsgemäßen Verbindungen der Formel I sind dadurch herstellbar, daß man ausgehend von einer Verbindung der Formel II

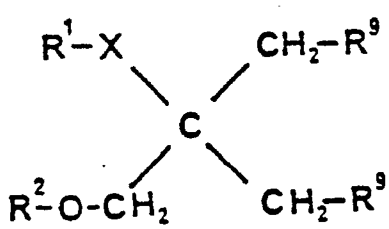

II

worin

$R^1$ ein Wasserstoffatom oder eine Alkylgruppe der Formel $CH_3(CH_2)_n$ mit n = 0 bis 5,
$R^2$ ein Wasserstoffatom,
$R^9$ eine Azidogruppe und
X eine Methylen- oder eine Carbamoylgruppe oder eine kovalente Bindung zwischen $R^1$ und dem C-2-Atom sind,

in an sich bekannter Weise ein Ether- oder Glycosid-Derivat der Formel II, worin die Reste $R^1$, $R^9$ und X ihre vorher genannte Bedeutung beibehalten und $R^2$ eine Alkylgruppe mit 1 bis 6 Kohlenstoffatomen, eine etherartig gebundene Gruppe der Formel $R^3\text{-O-CH}_2\text{-(CHR}^4)_m\text{-CH}_2\text{-}$, in der $R^3$ ein Wasserstoffatom oder eine Alkylgruppe mit 1 bis 6 Kohlenstoffatomen, $R^4$ eine Hydroxygruppe oder eine Alkyloxygruppe und m = 0 bis 2 sind, oder eine etherartig gebundene Gruppe der Formel $H\text{-}(CH_2)_a\text{-}(O\text{-}(CH_2)_b)_c\text{-}$ mit a = 0 bis 4, b = 1 bis 4 und c = 1 bis 7, oder ein 0-glycosidisch gebundener Rest der Formel

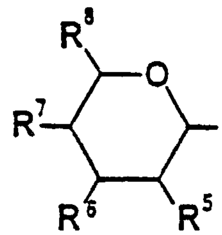

in dem

$R^5$, $R^6$ und $R^7$ unabhängig voneinander ein Wasserstoffatom oder eine Hydroxygruppe sind oder $R^5$ und $R^6$ für ein Elektronenpaar stehen und
$R^8$ ein Wasserstoffatom, eine Methyl- oder Hydroxymethylgruppe ist,

darstellt und das erhaltene Derivat in Gegenwart von Palladium/Kohle und eines organischen Lösungsmittels wie Methanol, Essigsäureethylester oder Dioxan hydriert, wobei eine Diaminoverbindung gebildet wird, die in an sich bekannter Weise mit $K_2PtCL_4$ zu einem Platinkomplex der Formel I umgesetzt wird, aus dem anschließend weitere Derivate der Formel I hergestellt werden.

Die Herstellung einer Verbindung der allgemeinen Formel I basiert auf der Anwendung von Verfahren, die zum Beispiel in der Offenlegungschrift DE 34 32 320 beschrieben wurden oder die in der Kohlenhydratchemie üblich sind.

Die Bestimmung ihrer zytostatischen Wirksamkeit erfolgte in vitro an L1210-Leukämiezellen der Maus oder in vivo an L1210-Leukämie, B16-Melanoma und Lewis Lung Adenocarcinoma. Die akute Toxizität der Verbindungen wurde an NMRI-Mäusen ermittelt. Unter Berücksichtigung der geringen akuten Toxizität (H.P. Kraemer, H.H. Sedlacek, Behring Institute Mitt. 74, 301-328, 1984) erweisen sich die erfindungsgemäßen Verbindungen dem Cisplatin überlegen hinsichtlich Zytotoxizität, Löslichkeit und Wirksamkeit an der L1210-Leukämie. Des weiteren sind die erfindungsgemäßen Verbindungen auch bei Tumorzellen mit Resistenz gegen Cisplatin wirksam.

Gegenstand der Erfindung sind auch Arzneimittel vorzugsweise zur Tumortherapie, die eine wirksame Menge einer oder mehrerer der Verbindungen der Formel I als Wirkstoff enthalten.

Die Dosierungs- und Anwendungs-Weise entspricht im wesentlichen der für Cis-$(NH_3)_2PtCl_2$ bekannten, wobei aber aufgrund des günstigen therapeutischen Indexes der erfindungsgemäßen Verbindungen auch höhere Dosierungen und/oder eine häufigere Verabreichung in Frage kommen.

Neben den üblichen pharmazeutischen Konfektionierungs-und/oder Verdünnungsmitteln können diese Arzneimittel neben den Verbindungen der Formel I zur Unterstützung der Therapie ggf. auch noch weitere Wirkstoffe enthalten, sofern diese mit den erfindungsgemäßen Verbindungen der Formel I zusammen keine unerwünschten Nebenwirkungen zeigen.

## Beispiele

In den folgenden Beispielen wird die vorliegende Erfindung näher beschrieben, ohne sie hierbei einzuschränken.

### Beispiel 1:

Herstellung von 1,3-Diazidopropan-Derivaten

2,2-Bis-(azidomethyl)-propan-1-ol (Verbindung 1)

150 g (1,25 mol) 2-Hydroxymethyl-2-methylpropan-1,3-diol wurden in 1,5 l Pyridin gelöst und mit 487 g (2,5 mol) p-Toluolsulfonsäurechlorid versetzt. Nach 18 h Rühren bei Raumtemperatur wurde der Reaktionsansatz im Wasserstrahlpumpenvakuum (i. Vak.) eingeengt und zweimal mit Toluol abdestilliert. Der Rückstand wurde in Chloroform aufgenommen und die Lösung wurde dreimal mit Eiswasser gewaschen. Die organische Phase wurde i. Vak. eingeengt und der Rückstand wurde chromatographisch über 2000 g Kieselgel (Elutionsmittel: Dichlormethan/Essigsäureethylester) gereinigt. Das resultierende Produkt (245 g) wurde in 1 l DMF gelöst und mit 76,5 g (1,17 mol) Natriumazid versetzt. Der Reaktionsansatz wurde 1 h bei 110°C gerührt. Die Reaktionsmischung wurde i. Vak. eingeengt und der Rückstand wurde in Essigsäureethylester gelöst und durch dreimaliges Ausschütteln mit Wasser wurden die wasserlöslichen Bestandteile entfernt. Die organische Phase wurde i. Vak. eingeengt und der resultierende Rückstand wurde säulenchromatographisch über 1500 g Kieselgel (Elutionsmittel: Petrolether/Toluol/Essigsäureethylester 20:10:1) gereinigt.
Ausbeute: 79,8 g (83 %)
IR ($cm^{-1}$, $N_3$) : 2100
$^{13}C$-NMR (90 MHz, $CDC1_3$, delta) : 65,58 ($CH_2OH$), 55,37 (2× $CH_2N$), 40,64 (C), 14,47 ($CH_3$).

2,2-Bis-(azidomethyl)-butan-1-ol (Verbindung 2)

Ausgehend von 2-Ethyl-2-hydroxymethyl-propan-1,3-diol wurde die Verbindung 2 nach der Vorschrift zur Synthese der Verbindung 1 hergestellt.
IR ($cm^{-1}$, $N_3$) : 2100.

2-Acetamido-2,2-bis-(azidomethyl)-ethanol (Verbindung 3)

100 g (0,82 mol) Tris-hydroxymethyl-aminomethan wurden in 600 ml Pyridin gelöst und bei -20°C mit 790 ml Acetanhydrid versetzt. Nach 10 h Rühren bei Raumtemperatur wurde der Reaktionsansatz wie üblich aufgearbeitet. Das resultierende Tetraacetylderivat (181 g) wurde in Methanol gelöst und mit einer katalytischen Menge Natriummethylat bei 0°C versetzt. Nach 3 h wurde mit DOWEX WX8 neutralisiert und zur Trockne eingeengt. Das Produkt: Trishydroxymethyl-acetamidomethan wurde nach der Vorschrift zur Herstellung der Verbindung 1 zur Verbindung 3 umgesetzt.
IR ($cm^{-1}$) : 2100, 1660.
$^{13}C$-NMR (90 MHz, $CDC1_3$, delta) : 168,18 (CONH), 75,04 (C), 72,65 ($CH_2OH$), 56,24 (2×$CH_2N_3$), 14,35 ($CH_3$).

Beispiel 2:

Alkylierung und Glycosidierung von Verbindungen 1 und 2.

2,2-Bis-(azidomethyl)-propyl-methylether (Verbindung 4)

7,3 g (43 mmol) Verbindung 1 wurden in 50 ml Dioxan gelöst und mit 7,2 g Kalium-tert.-butylat versetzt. In die Mischung wurden 6,71 g (47 mmol) Methyljodid, gelöst in 20 ml Dioxan, getropft. Nach 3 h Rühren bei Raumtemperatur wurde der Reaktionsansatz i. Vak. eingedampft. Der resultierende Sirup wurde chromatographisch über 70 g Kieselgel (Elutionsmittel: Hexan/Diisopropylether 5:1) gereinigt.
Ausbeute: 6,3 g (80 %).
Elementaranalyse:
Ber. C 39,12 H 6,56 N 45,62
Gef. C 39,06 H 6,52 N 45,32
$^{13}C$-NMR (90 MHz, $CDC1_3$,delta): 75,74 ($CH_2O$), 59,65 ($CH_3O$), 56,29 (2× $CH_2N_3$), 41,61 (C), 18,96 ($CH_3$).

2,2-Bis-(azidomethyl)-butyl-methylether (Verbindung 5)

Ausgehend von Verbindung 2 wurde die Verbindung 5 nach der Vorschrift zur Synthese von Verbindung 4 hergestellt.
IR ($cm^{-1}$, $N_3$) : 2100.
$^{13}C$-NMR (90 MHz, $CDCl_3$,delta): 72,76 ($CH_2O$), 58,89 ($CH_3O$), 53,20 (2× $CH_2N_3$), 43,12 (C), 23,40 ($CH_2$),

7,1 ($CH_3$).

2,2-Bis-(azidomethyl)-propyl-(2',3'-dihydroxypropyl)-ether (Verbindung 6)

15 g (88 mmol) Verbindung 1 wurden in 200 ml DMF gelöst und mit 9,9 g (176 mmol) KOH versetzt. 24 g (176 mmol) Epibromhydrin, gelöst in 50 ml DMF, wurden innerhalb von 1 h zugetropft. Nach 24 h Rühren bei Raumtemperatur wurde der Reaktionsansatz abfiltriert und zum Sirup i. Vak. eingeengt. Das Produkt wurde noch chromatographisch über 500 g Kieselgel (Elutionsmittel: Dichlormethan/Petrolether/Essigsäureethylester 1:1:0,1) gereinigt. Das resultierende Glycidylether-Derivat wurde in Dioxan/Wasser 1:1 emulgiert und mit KOH bei 70° C zur Verbindung 6 hydrolysiert. Nach Neutralisation mit Salzsäure wurde der Ansatz eingeengt, in Essigsäureethylester gelöst und mit 20 g Natriumsulfat gerührt. Nach der Filtration und Abdampfen der organischen Phase wurde der Rückstand chromatographisch (200 g Kieselgel, Elutionsmittel: Dichlormethan/Aceton 6:1) gereinigt.
Ausbeute: 16,3 g (76 %).
$^{13}$C-NMR (90 MHz, $CDCl_3$,delta) : 74,55 ($CH_2$), 73,25 ($CH_2$), 71,24 (CHOH), 64,42 ($CH_2OH$), 56,29 (2×$CH_2N_3$), 41,55 (C), 19,07 ($CH_3$).
IR ($cm^{-1}$, $N_3$) : 2100.

2,2-Bis-(azidomethyl)-propyl-(2'-hydroxy-3'-methoxypropyl)-ether (Verbindung 7)

Das Glycidylether Derivat (4 g), wie bei der Herstellung der Verbindung 6 beschrieben, wurde in trockenem Methanol gelöst und mit festem KOH (2 g) bei Raumtemperatur gespalten. Die resultierende Methoxy-Verbindung 7 wurde anschließend über Kieselgel filtriert.
Ausbeute: 4,3 g (99 %)
IR ($cm^{-1}$, $N_3$) : 2100.
$^{13}$C-NMR (90 MHz), $CDCl_3$,delta) : 74,44 (2× $CH_2$), 73,14 (CH), 69,83 ($CH_2$), 59,76 ($OCH_3$), 56,29 (2× $CH_2N_3$), 41,66 (C), 19,07 ($CH_3$).

2,2-Bis-(azidomethyl)-propyl-2,3-didesoxy-alpha-D-erythrohex-2-enopyranosid (Verbindung 8) und 2,2-Bis-(azidomethyl)-propyl-2-desoxy-alpha-D-arabinohexopyranosid (Verbindung 9)

7,5 g (44 mmol) Verbindung 1 wurden in 250 ml Dioxan aufgenommen. Nach der Zugabe von 12 g (44 mmol) 3,4,6-Tri-O-acetyl-D-glucalund 0,960 g (4,4 mmol) p-Toluolsulfonsäure wurde der Reaktionsansatz 20 h bei 70° C gerührt. Dann wurde eingeengt und wie üblich aufgearbeitet. Durch eine säulenchromatographische Trennung wurden zwei Verbindungen erhalten: 2,2-Bis-(azidomethyl)-propyl-4,6-di-O-acetyl-2,3-didesoxy-alpha-D-erythrohex-2-enopyranosid (7 g = 40 %) und 2,2-Bis-(azidomethyl)-propyl-3,4,6-tri-O-acetyl-alpha-D-arabinohexopyranosid (3,5 g = 21 %). Beide Verbindungen wurden mit Natriummethylat, wie üblich, entacetyliert.

Verbindung 8:

Ausbeute: 3,2 g
IR ($cm^{-1}$, $N_3$) : 2100.
$^{13}$C-NMR (90 MHz, $CDCL_3$,delta) : 134,09 (CH; C-2), 126,18 (CH; C-3), 95,08 (CH C-1), 72,22 ($CH_2$), 71,19 (CH), 64,20 ($CH_2$), 62,79 (CH), 56,18 (2× $CH_2N_3$), 41,28 (C), 19,02 ($CH_3$).

Verbindung 9:

Ausbeute 2,7 g
IR ($cm^{-1}$, $N_3$) : 2100
$^{13}$C-NMR (90 MHz, $CDCl_3$,delta) : 98,72 (CH, C-1), 72,54 (CH), 72,38 (CH), 70,54 (CH), 69,68 ($CH_2$), 62,25 ($CH_2$), 56,19 (2× $CH_2N_3$), 41,28 ($CH_2$), 37,92 (C), 19,23 ($CH_3$)
$^{1}$H-NMR (90 MHz, $CDCl_3$,delta) : 4,78 (1-H;J(1,2) = 1,2 Hz, J (1,2') = 2 Hz).

2,2-Bis-(azidomethyl)-propyl-β-D-glucopyranosid(Verbindung 10)

3,75 g (22 mmol) Verbindung 1 wurden in 60 ml Toluol/Nitromethan 1:1 gelöst. Nach der Zugabe von 5,5 g (72 mmol) Quecksilbercyanid und 9,1 g (22 mmol) Acetobromglucose wurde die Reaktionsmischung

auf 40 ° C erwärmt, wobei unter reduziertem Druck 10 Volumen % der Reaktionslösung abdestilliert wurde.

Nach 14 h wurde der Ansatz mit Chloroform versetzt, zweimal mit einer 10 %igen KJ-Lösung, einmal 1 %iger Natriumhydrogencarbonat-Lösung und einmal mit Eiswasser gewaschen. Nach Trocknen mit Natriumsulfat wurde i. Vak. eingeengt und der Rückstand wurde säulenchromatographisch gereinigt. Die resultierende Verbindung wurde in Gegenwart von Natriummethylat entacetyliert.

Ausbeute: 5,9 g (82 %)

Elementaranalyse:

Ber: C 39,75 H 6,06 N 25,29

Gef: C 39,82 H 6,05 N 25,13

IR ($cm^{-1}$, $N_3$) : 2100

Beispiel 3:

Herstellung von Diaminoliganden und deren Komplexierung mit Kaliumtetrachloroplatinat

2-Methoxymethyl-2-methyl-1,3-propan-diamin-N,N'-platin(II)-dichlorid (Verbindung 11)

4,8 g (26,05 mmol) Verbindung 4 wurden in 30 ml einer Mischung aus Methanol und Essigsäureethylester 3:1 gelöst. Nach der Zugabe von 3 g Pd/C (10 %ig) wurde der Reaktionsansatz unter Rühren 2 h bei Raumtemperatur hydriert. Der Verlauf der Hydrierung wurde dünnschichtchromatographisch verfolgt. Nach Abfiltrieren des Katalysators wurde die Lösung zur Trockne eingedampft. Das resultierende Produkt, das im IR-Spektrum keine Azidobande zeigt, wurde ohne weitere Reinigungsschritte in die folgende Umsetzung mit Platin-Salzen eingesetzt.

Ausbeute: 3 g (88 %).

3 g (22,7 mmol) Diaminozwischenverbindung wurden in Methanol gelöst und zu 50 ml einer wäßrigen Lösung aus 9,4 g (22,7 mmol) $K_2PtCl_4$ gegeben. Nach 18 h Rühren bei Raumtemperatur wurde das ausgefallene Reaktionsprodukt abfiltriert und mehrere Male mit Eiswasser gewaschen. Die vereinigten Filtrate wurden auf ein Volumen von 15 ml i. Vak. eingedampft, wobei erneut das Reaktionsprodukt ausgefallen war.

Ausbeute: 6 g (68 %)

Elementaranalyse:

Ber: C 18,10 H 4,05 Cl 17,8 N 7,03 Pt 49,0

Gef. C 18,50 H 4,00 Cl 17,3 N 6,91 Pt 48,5

IR ($cm^{-1}$) : 3500, 3250, 3150, 2950, 2930, 2880, 2770, 1575, 1450.

2-Ethyl-2-methoxymethyl-1,3-propan-diamin-N,N'-platin(II)-dichlorid (Verbindung 12)

Die Verbindung 12 wurde nach der Vorschrift zur Synthese der Verbindung 11 hergestellt.

9,3 g (46,5 mmol) Verbindung 5 wurden hydriert und das Diaminozwischenprodukt wurde mit 19,3 g (46,5 mmol) $K_2PtCl_4$ zur Verbindung 12 umgesetzt.

Elementaranalyse:

Ber: C 20,40 H 4,40 Cl 17,19 N 6,79 Pt 47,32

Gef: C 20,24 H 4,61 Cl 17,50 N 6,50 Pt 46,80

2-(2',3'-Dihydroxypropyloxymethyl)-2-methyl-1,3-propandiamin-N,N'-platin(II)-dichlorid (Verbindung 13)

Die Verbindung 13 wurde nach der Vorschrift zur Synthese der Verbindung 11 hergestellt.

7,5 g (30,7 mmol) Verbindung 6 wurde hydriert und das resultierende Diaminozwischenprodukt wurde mit 12,7 g (30,7 mmol) $K_2PtCl_4$ zur Verbindung 13 umgesetzt.

Ausbeute: 7,2 g (52 %)

Elementaranalyse:

Ber: C 20,69 H 4,39 Cl 15,47 N 6,14 Pt 42,57

Gef: C 20,32 H 4,21 Cl 15,17 N 6,01 Pt 41,73

$^{13}C$-NMR (90 MHz, $D_2O$,delta, Standard Dioxan) : 80,63, 74,48, 72,39, 65,08, 52,79 ($CH_2NH_2$), 39,37, 20,95.

2-(2'-Hydroxy-3'-methoxypropyloxymethyl)-2-methyl-1,3-propandiamin-N,N'-platin(II)-dichlorid (Verbindung 14)

Die Verbindung 14 wurde ausgehend von Verbindung 7 nach dem Verfahren zur Synthese der Verbindung 11 hergestellt.
Elementaranalyse:
Ber: C 22,88 H 4,69 Cl 15,01 N 5,93 Pt 41,30
Gef: C 23,40 H 4,61 Cl 15,20 N 5,81 Pt 41,10
$^{13}$C-NMR (90 MHz, $D_2O$,delta, Standard Dioxan) : 80,96, 75,56, 74,48, 70,48, 60,64, 52,70 (2× $CH_2NH_2$), 39,05, 21,27.

2-(2',3'-Didesoxy-alpha-D-erythrohexopyranosyloxymethyl)-2-methyl-1,3-propan-diamin-N,N'-platin(II)-dichlorid (Verbindung 15)

9 g (30,30 mmol) Verbindung 8 wurden in Gegenwart von 4 g Pd/C (10 %) und 200 ml Methanol hydriert. Die resultierende Verbindung wurde mit 12,0 g (30,30 mmol) $K_2PtCl_4$, wie bereits oben beschrieben, zur Verbindung 15 umgesetzt.
Ausbeute: 7 g (45 %)
$^{13}$C-NMR (90 MHz, DMF,delta) : 97,46, 76,55, 73,08, 67,18, 63,50, 50,98, 50,60, 39,44, 30,99, 28,39, 19,93.

2-(β-D-Glucopyranosyloxymethyl)-2-methyl-1,3-propandiamin-N,N'-platin(II)-dichlorid (Verbindung 16)

3,32 g (10 mmol) Verbindung 10 wurden in Gegenwart von Pd/C hydriert und das resultierende Diamin wurde mit $K_2PtCl_4$ zur Verbindung 16 umgesetzt.
Ausbeute: 4,9 g (89 %)
Elementaranalyse:
Ber: C 24,17 H 4,42 Cl 12,97 N 5,12 Pt 35,72
Gef: C 24,03 H 4,41 Cl 12,38 N 5,01 Pt 35,20

2-Acetamido-2-hydroxymethyl-1,3-propan-diamin-N,N'-platin(II)-dichlorid (Verbindung 17)

2,8 g (14,07 mmol) Verbindung 3 wurden in Gegenwart von Pd/C hydriert und das Diaminoprodukt wurde mit $K_2PtCl_4$ zur Verbindung 17 umgesetzt.
Ausbeute 3,19 g (55 %)
Elementaranalyse:
Ber: C 16,86 H 3,54 Cl 16,59 N 9,83 Pt 45,66
Gef: C 17,00 H 3,50 Cl 16,60 N 9,90 Pt 46,10
$^{13}$C-NMR (90 MHz, DMF,delta) : 169,79 (CO), 83,84 ($CH_2OH$), 64,20 (C), 51,11 ($CH_2N$), 48,78 ($CH_2N$), 14,82 ($CH_3$).

Beispiel 4:

Die Verbindungen 11 bis 17, die als Platin (II)-dichlorid-Derivate vorliegen, wurden nach dem folgenden Schema in ihrer Nitrat-, Hydroxy- und Carbonsäure-Derivate umgesetzt.

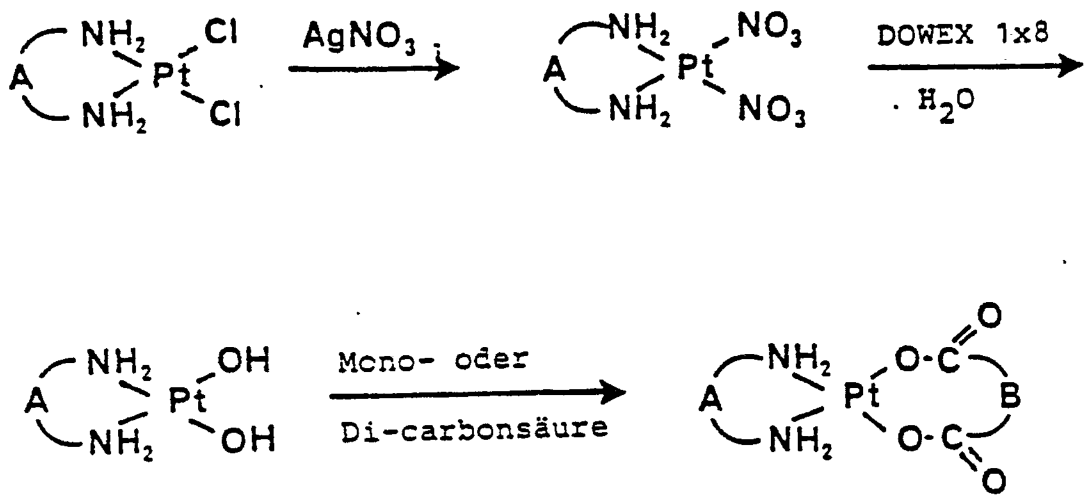

a) Herstellung von Platin-Komplexen mit Nitrat als Liganden

10 mmol Platin(II)-dichlorid-Derivat wurden in 100 ml destilliertem entgastem Wasser suspendiert. Nach der Zugabe von 20 mmol Silbernitrat, gelöst in 50 ml Wasser, wurde der Reaktionsansatz 25 h bei Raumtemperatur unter Lichtausschluß gerührt. Der Reaktionsablauf wurde dünnschichtchromatographisch auf Cellulose (13255 Fa. Eastman, Laufmittel: Butanol/Eisessig/Wasser 5:3:2) sowie mittels HPLC-Technik (RP18 Lichrosorb 7 $\mu$ 250×4, Elutionsmittel: Methanol/Wasser-Gradient, Detektion: UV 220 nm) verfolgt. Nach Abfiltrieren des ausgefallenen Silberchlorids wurde das in Wasser gelöste Nitrat-Derivat ohne weitere Reinigungsschritte in die folgende Umsetzung eingeleitet.

b) Herstellung von Platin-Komplexen mit Hydroxy-Gruppen als Liganden

Die Lösung mit dem Dinitratzwischenprodukt wurde unter Rühren mit 10 g Ionenaustauschharz (DOWEX, Type 1×8; Akitivierung mit 10n NaOH) versetzt. Nach 30 min zeigte das Dünnschichtchromatogramm (n-Butanol/Eisessig/Wasser 5:3:2; Cellulose-Folie (13255 Fa. Eastmann)), daß der Austausch des Nitrates durch Hydroxygruppen vollständig abgelaufen war. Nach Abfiltrieren des Harzes wurde das Filtrat zur Trockne unter Lichtausschluß i. Vak. eingeengt.

c) Herstellung von Platin-Komplexen mit Carbonsäure als Ligand bzw. Liganden

10 mmol Platin(II)-dihydroxy-Verbindung wurden in 50 ml destilliertem, entgastem Wasser gelöst. Zu dieser Lösung wurden 10 mmol Di- oder Oligocarbonsäure, gelöst in 20 ml Wasser, unter Rühren und Lichtausschluß gegeben. Nach 6 h wurde die Reaktionslösung i. Vak. zur Trockne eingedampft. Das Produkt wurde aus Wasser/Methanol umkristallisiert.

Wie in Beispiel 4a beschrieben, wurden die folgenden Verbindungen hergestellt:

2-Methoxymethyl-2-methyl-1,3-propan-diamin-N,N'-platin (II)-dinitrat (Verbindung 18)

Ausgehend von Verbindung 11 wurde die Verbindung 18 hergestellt.

2-Ethyl-2-methoxymethyl-1,3-propan-diamin-N,N'-platin(II)-dinitrat (Verbindung 19)

Ausgehend von Verbindung 12 wurde die Verbindung 19 hergestellt.

2-(2',3'-Dihydroxypropyloxymethyl)-2-methyl-1,3-propandiamin-N,N'-platin(II)-dinitrat (Verbindung 20)

Ausgehend von Verbindung 13 wurde die Verbindung 20 hergestellt.

2-(2'-Hydroxy-3'-methoxy-propyloxymethyl)-2-methyl-1,3-propan-diamin-N,N'-platin(II)-dinitrat (Verbindung 21)

Ausgehend von Verbindung 14 wurde die Verbindung 21 hergestellt.

2-(2',3'-Didesoxy-alpha-D-erythrohexopyranosyloxymethyl)-2-methyl-1,3-propan-diamin-N,N'-platin(II)-dinitrat (Verbindung 22)

Ausgehend von Verbindung 15 wurde die Verbindung 22 hergestellt.

2-($\beta$-O-Glucopyranosyloxymethyl)-2-methyl-1,3-propandiamin-N,N'-platin(II)-dinitrat (Verbindung 23)

Ausgehend von Verbindung 16 wurde die Verbindung 23 hergestellt.
Wie in Beispiel 4b beschrieben, wurden folgende Verbindungen hergestellt:

2-Methyloxymethyl-2-methyl-1,3-propan-diamin-N,N'-platin (II)-hydroxyd (Verbindung 24)

Ausgehend von Verbindung 18 wurde die Verbindung 24 hergestellt.
Elementaranalyse:
Ber: C 19,93 H 5,01 N 7,75 Pt 54,02

Gef: C 19,57 H 5,06 N 7,37 Pt 53,72.

2-Ethyl-2-methoxymethyl-1,3-propan-diamin-N,N'-platin (II)hydroxyd (Verbindung 25)

Ausgehend von Verbindung 19 wurde die Verbindung 25 hergestellt. HPLC (RP-8, 7μ, Wasser, UV 220 nm), Rt-time: 2,34 min.
Elementaranalyse:
Ber: C 22,37 H 5,36 N 7,46 Pt 51,96
Gef: C 22,18 H 5,38 N 7,22 Pt 51,36.

2-(2',3'-Dihydroxypropyloxymethyl)-2-methyl-1,3-propan-diamin-N,N'-platin(II)-hydroxyd (Verbindung 26)

Ausgehend von Verbindung 20 wurde die Verbindung 26 hergestellt.
Elementaranalyse:
Ber: C 22,79 H 5,26 N 6,64 Pt 46,31
Gef: C 22,61 H 5,17 N 6,51 Pt 45,95.

2-(2'-Hydroxy-3'-methoxy-propyloxymethyl)-2-methyl-1,3-propan-diamin-N,N'-platin(II)-hydroxyd (Verbindung 27)

Ausgehend von Verbindung 21 wurde die Verbindung 27 hergestellt.
Elementaranalyse:
Ber: C 24,82 H 5,55 N 6,43 Pt 44,82
Gef: C 24,79 H 5,58 N 6,32 Pt 44,37.

2-(2',3'-Didesoxy-alpha-D-erythrohexopyranosyloxymethyl)-2-methyl-1,3-propan-diamin-N,N'-platin(II)-hydroxyd (Verbindung 28)

Ausgehend von Verbindung 22 wurde die Verbindung 28 hergestellt.
Elementaranalyse:
Ber: C 27,66 H 5,48 N 5,86 Pt 40,87
Gef: C 27,73 H 5,47 N 5,83 Pt 40,61

2-(β-O-Glucopyranosyloxymethyl)-2-methyl-1,3-propan-diamin-N,N'-platin(II)-hydroxyd (Verbindung 29)

Ausgehend von Verbindung 23 wurde die Verbindung 29 hergestellt.
Elementaranalyse:
Ber: C 25,92 H 5,14 N 5,50 Pt 38,31
Gef: C 25,81 H 5,03 N 5,31 Pt 38,09.

Wie im Beispiel 4c beschrieben wurden folgende Verbindungen hergestellt:

2-Methyloxymethyl-2-methyl-1,3-propan-diamin-N,N'-platin(II)-malonat (Verbindung 30)

Ausgehend von Verbindung 24 wurde die Verbindung 30 hergestellt. HPLC (RP-8, 7μ, Elutionsmittel: Wasser/Methanol 85:15, UV 220 nm) : Rt-time 5,12 min.
$^{13}$C-NMR (90 MHz, $D_2O$,delta) : 181,34 (COO), 81,39 ($CH_2$), 61,34 ($CH_3O$, 53,02 (2× $CH_2N$), 50,16 ($CH_2$), 40,45 (C), 20,76 ($CH_3$).

2-Methyloxymethyl-2-methyl-1,3-propan-diamin-N,N'-platin (II)- 1,1-cyclobutandicarboxylat (Verbindung 31)

Ausgehend von Verbindung 24 wurde die Verbindung 31 hergestellt. HPLC (RP-8, 7μ, Wasser, UV 220 nm) : Rt-time 7,7 min.
$^{13}$C-NMR (90 MHz, $D_2O$,delta) : 184,78 (COO), 81,38 ($CH_2$), 61,72 ($CH_3$), 58,80 (C), 53,35 (2× $CH_2N$), 40,64 (C), 33,78 ($CH_2$), 33,33 ($CH_2$), 20,95 ($CH_3$), 17,78 ($CH_2$).
Elementaranalyse:
Ber: C 30,69 H 4,72 N 5,96 Pt 41,57
Gef: C 30,87 H 4,83 N 5,71 Pt 41,08

2-Ethyl-2-methyloxymethyl-1,3-propan-diamin-N,N'-platin (II)-1,1-cyclobutandicarboxylat (Verbindung 32)

Ausgehend von Verbindung 25 wurde die Verbindung 32 hergestellt. HPLC (RP-8, 7µ, Wasser/Methanol 95:5, UV 220) : Rt-time 17,61 min.
$^{13}$C-NMR (90 MHz, $D_2O$,delta) : 184,77 (COO), 79,37 ($CH_2$), 61,91 ($CH_2$), 59,37 (C), 51,81 ($CH_2N$), 51,69 ($CH_2N$), 43,30 (C), 34,16 ($CH_2$), 33,97 ($CH_2$), 27,81 ($CH_3$), 18,41 ($CH_2$), 9,46 ($CH_3$).
Elementaranalyse:
Ber: C 35,85 H 5,50 N 6,43 Pt 44,80
Gef: C 36,03 H 5,52 N 6,32 Pt 44,37.

2-(2',3'-Dihydroxypropyloxymethyl)-2-methyl-1,3-propan-diamin-N,N'-platin(II)-N-(carbamoylmethyl)-iminodiacetat (Verbindung 33)

Ausgehend von Verbindung 26 wurde die Verbindung 33 hergestellt.
Elementaranalyse:
Ber: C 29,21 H 4,90 N 9,73 Pt 33,20
Gef: C 29,23 H 4,91 N 9,63 Pt 32,97.

2-(2'-Hydroxy-3'-methoxy-propyloxymethyl)-2-methyl-1,3-propandiamin-N,N'-platin(II)-1,1-cyclobutandicarboxylat (Verbindung 34)

Ausgehend von Verbindung 28 wurde die Verbindung 34 hergestellt.
Elementaranalyse:
Ber: C 33,14 H 5,19 N 5,15 Pt 35,90
Gef: C 32,06 H 5,07 N 5,12 Pt 35,63

2-(β-O-Glucopyranosyloxymethyl)-2-methyl-1,3-propan-diamin-N,N'-platin(II)-malonat (Verbindung 35)

Ausgehend von Verbindung 29 wurde die Verbindung 35 hergestellt.
Elementaranalyse:
Ber: C 29,11 H 4,53 N 4,85 Pt 33,80
Gef: C 29,05 H 4,50 N 4,73 Pt 33,22.

Beispiel 5:

Ermittlung der zytostatischen Aktivität

Die Bestimmung der zytostatischen Wirksamkeit der erfindungsgemäßen Verbindungen erfolgte an L1210-Leukämiezellen der Maus. Im einzelnen wurden folgende Testsysteme verwendet:

a) Koloniebildung von L1210-Leukämiezellen in soft agar

Diese Methode dient zum Nachweis eines Einflusses der Testsubstanzen auf das Wachstumsverhalten der Zellen über mehrere Generationen (bei einer Zellzykluszeit von 10-12 Stunden werden in der Testzeit von 7 Tagen etwa 14 aufeinanderfolgende Generationen beobachtet). Zytostatisch wirksame Substanzen bewirken in diesem Test eine Reduktion der zu beobachtenden Koloniezahl gegenüber einer unbehandelten Kontrolle. Im einzelnen wird der Test wie folgt durchgeführt.
500 Leukämiezellen pro Platte werden mit unterschiedlichen Konzentrationen von Testsubstanz 1 Stunde bei 37 °C inkubiert. Anschließend werden die Zellen zweimal mit McCoy5A-Medium gewaschen und schließlich in Petrischalen nach Zusatz von 0,3 % Agar ausgegossen. Kontrollen werden lediglich mit frischem Medium inkubiert. Anstelle der einstündigen Inkubation werden in manchen Fällen unterschiedliche Konzentrationen und Testsubstanzen der oberen Agarschicht zugemischt, um so eine kontinuierliche Exposition der Zellen über die gesamte Inkubationszeit zu erreichen. Nach Erstarren des Agars werden die Platten im Brutschrank 7 Tage bei 37 °C inkubiert (5 Vol-% $CO_2$, 95 % relative Luftfeuchtigkeit). Anschließend wird die Anzahl der entstandenen Kolonien mit einem Durchmesser von mehr als 60 µ gezählt. Die Ergebnisse werden angegeben als Koloniezahl in behandelten Agarplatten, in Prozent der unbehandelten Kontrolle. Aus der so erhaltenen Dosiswirkungskurve wird die $IC_{50}$ als Maß für die Wirksamkeit der Substanz ermittelt.

Die Ergebnisse der Prüfungen im Vergleich zu Cisplatin sind in der Tabelle 1 zusammengefaßt.

b) Ermittlung der akuten Toxizität

Zur Ermittlung der akuten Toxizität werden NMRI-Mäusen am Tag 0 unterschiedliche Dosen der Testsubstanz, gelöst in 0,5 ml 5 %iger Glucose-Lösung, intraperitoneal injiziert. Kontrollgruppen erhalten lediglich 0,5 ml 5 %ige Glucose-Lösung. Pro Konzentration der Testsubstanz werden 5 Mäuse verwendet. Am Tag 14 wird die Zahl der überlebenden Mäuse ermittelt und daraus nach der Lichtfield Wilcoxon Methode die LD5, LD50 und LD95 ermittelt. Die Toxizität ($LD_{50}$ (mg/kg)) der hier beschriebenen Verbindungen im Vergleich zu Cisplatin ist in Tabelle 1 zusammengefaßt.

c) in vivo Wirksamkeit der Platinkomplexe gegen L1210-Leukämie der Maus

Methodik:

Ascitesflüssigkeit wird unter sterilen Bedingungen DBA2-Mäusen (weiblich, 18-20 g) 7 Tage nach Implantation entnommen. Der Ascites wird dreimal mit PBS gewaschen, gezählt und auf eine Zellzahl von $10^6$ in 0,2 ml PBS eingestellt.
$10^6$ Zellen, suspendiert in 0,2 ml PBS, werden anschließend DBF1-Mäusen (weiblich, 18-20 g) intraperitoneal injiziert. 6 Tiere pro Gruppe werden für jede Substanzkonzentration bzw. als Kontrolle eingesetzt.

Ermittlung der antitumoralen Wirksamkeit:

a) Die Tiere werden am Tag 1 und 5 nach Injektion der Testsubstanz gewogen. Gewichtsverlust von mehr als 20 % am Tag 5 wird als Indikator einer toxischen Substanzwirkung angesehen.
b) Am Ende des Experimentes (Tod aller Tiere oder überlebende Tiere am Tag 60) wird die mittlere Überlebenszeit der Tiere in den jeweiligen Gruppen bestimmt, sofern am Tag 5 des Experimentes mindestens 65 % der Tiere noch gelebt hatten. Die mittlere Überlebenszeit wird ausschließlich für im Verlaufe des Experimentes sterbenden Tiere bestimmt. Langzeitüberlebende (LTS) werden bei dieser Berechnung nicht berücksichtigt und gesondert aufgeführt.
Aus der mittleren Überlebenszeit (MST) der behandelten Gruppen sowie der Kontrollgruppen ($MST_c$) wird die antitumorale Wirksamkeit (T/C) für die jeweilige Substanzkonzentration in Prozent der unbehandelten Kontrolle entsprechend der folgenden Formel bestimmt:

$$T/C\ \% = \frac{MST_T}{MST_C} \times 100$$

T/C-Werte größer als 125 % werden als Indikator einer signifikanten antitumoralen Wirksamkeit der Testsubstanz angesehen. Die Dosis, die den größten antitumoralen Effekt verursacht (optimale Dosierung) sowie jeweils eine Dosisstufe oberhalb und unterhalb dieser Dosis werden in Tabelle 1 zusammengefaßt. Tiere, die am Tag 60 des Experimentes noch leben, werden als "Longtermsurvivors" getrennt aufgeführt.

Behandlungsschema:

Das in unterschiedlichen Experimenten jeweils eingesetzte Behandlungsschema ist in Tabelle 1 angegeben.

Tabelle 1:

| | a | b | c | |
|---|---|---|---|---|
| Verbindung Nr.: | $IC_{50}$ (µg/ml) | $LD_{50}$ (mg/kg); 1 x i.p. | Behandlungsschema | T/C % (opt. Dose/LTS*) L1210 |
| 11 | 1,4 | - | - | - |
| 12 | 0,5 | 19 | 3x i.p./i.p. | 227 (3/-) |
| 13 | 1,25 | 57,3 | 5x i.p./i.p. | 156 (8/-) |
| 14 | 0,75 | 57,1 | 3x i.p./i.p. | 168 (4/-) |
| 15 | 2,9 | 1 - 5 | - | - |
| 17 | 1,1 | 50 - 100 | 3x i.p./i.p. | 113 (30/-) |
| 30 | 1,4 | - | - | - |
| 31 | 2,6 | 250 | 2x i.p./i.p. | 226(112/3/6) |
| 32 | 3,6 | 437 | 5x i.p./i.p. | 194 (47,4/-) |
| Cisplatin | 0,04 | 14 | 3x i.p./i.p. | 157 (4/-) |

LTS* = Long Time Survivor

Die Löslichkeit der erfindungsgemäßen Platinkomplexe ist im Vergleich zu cis-Platin-(II)-diamin-dichlorid in der Tabelle 2 angegeben.

Tabelle 2:

| Verbindung Nr. | Löslichkeit im Wasser (mg/ml) |
|---|---|
| 11 | 3 |
| 13 | 25 |
| 14 | 16 |
| 15 | 17 |
| 17 | 10 |
| 30 | 18 |
| 31 | 15 |
| 32 | 70 |
| cis-platin-(II)-diamin-dichlorid | 1 |

**Patentansprüche**

**Patentansprüche für folgende Vertragsstaaten : AT, BE, CH, DE, FR, GB, IT, LI, LU, NL, SE**

**1.** Verbindung der allgemeinen Formel I

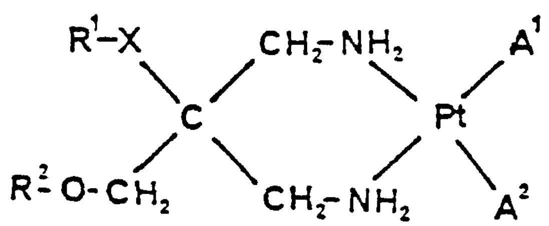

worin

$R^1$ ein Wasserstoffatom oder eine Alkylgruppe der Formel $CH_3(CH_2)_n$- mit n = 0 bis 5,

$R^2$ ein Wasserstoffatom, wenn X eine Carbamoylgruppe ist, eine Alkylgruppe mit 1 bis 6 Kohlenstoffatomen, eine etherartig gebundene Gruppe der Formel $R^3$-O-$CH_2$-$(CHR^4)_m$-$CH_2$-, in der

$R^3$ ein Wasserstoffatom oder eine Alkylgruppe mit 1 bis 6 Kohlenstoffatomen,

$R^4$ eine Hydroxygruppe oder eine Alkyloxygruppe mit 1 bis 3 Kohlenstoffatomen und m = O bis 2 sind, und

$R^2$ weiter eine etherartig gebundene Gruppe der Formel H-$(CH_2)_a$-(O-$(CH_2)_b)_c$- mit a = 0 bis 4, b = 1 bis 4 und c = 1 bis 7 oder einen O-glycosidisch gebundenen Rest der Formel

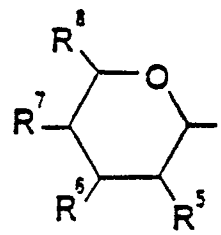

in dem

- $R^5$, $R^6$ und $R^7$ unabhängig voneinander ein Wasserstoffatom oder eine Hydroxygruppe und
- $R^8$ ein Wasserstoffatom, eine Methyl- oder Hydroxymethylgruppe sind,
- X eine Methylen- oder eine Carbamoylgruppe oder eine kovalente Bindung zwischen $R^1$ und dem C-2-Atom,
- $A^1$ und $A^2$ gleich sind und die Hydroxygruppe, Chlorid, Bromid, Jodid, Nitrat, Acetat oder Trifluoracetat oder
- $A^1$ Sulfat oder Carbonat und $A^2$ $H_2O$ oder
- $A^1$ und $A^2$ zusammen das Dianion einer organischen Säure wie Oxal-, Malon-, Hydroxymalon-, Ethylmalon-, 1,1-oder 1,2-Cyclobutan-dicarbon-, Phthal-, 3- oder 4-Carboxyphthal-, 3,4-Dicarboxyphthal oder N-(Carbamoylmethyl)-iminodiessigsäure darstellen.

2. Verbindung der allgemeinen Formel II, die als Vorprodukt zur Herstellung der Verbindung nach Anspruch 1 dient,

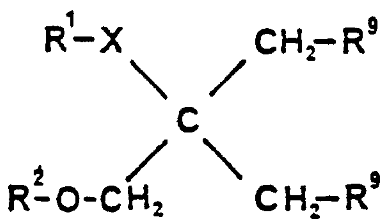

II

worin

- $R^1$ ein Wasserstoffatom oder eine Alkylgruppe der Formel $CH_3(CH_2)_n$ mit $n = 0$ bis 5,
- $R^2$ ein Wasserstoffatom
- $R^9$ eine Azidogruppe und
- X eine Methylen- oder eine Carbamoylgruppe oder eine kovalente Bindung zwischen $R^1$ und dem C-2-Atom sind.

3. Verbindung der allgemeinen Formel II, die als Zwischenprodukt zur Herstellung der Verbindungen nach Anspruch 1 dient,

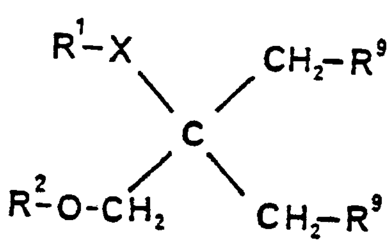

II

worin

- $R^1$, $R^2$ und X die Bedeutung nach Anspruch 1 haben und
- $R^9$ eine Azido oder eine Aminogruppe, die ggf. als Ammoniumsalz vorliegt, bedeuten.

4. Verfahren zur Herstellung einer Verbindung nach Anspruch 1, dadurch gekennzeichnet, daß man eine

Diamino-Verbindung der Formel II nach Anspruch 3 in an sich bekannter Weise mit $K_2PtCl_4$ zu einem Platinkomplex der Formel I umsetzt, aus dem anschließend weitere Derivate der Formel I hergestellt werden.

5. Verwendung einer Verbindung nach Anspruch 1 zur Herstellung eines Arzneimittels.

**Patentanspruch für folgende Vertragsstaaten : ES, GR**

1. Verfahren zur Herstellung einer Verbindung der allgemeinen Formel I

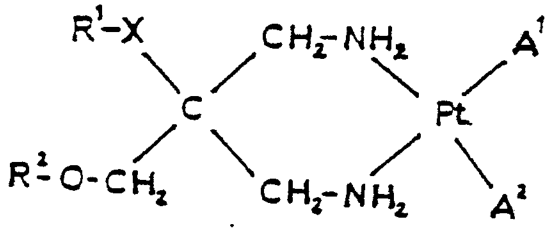

I

worin

- $R^1$ ein Wasserstoffatom oder eine Alkylgruppe der Formel $CH_3(CH_2)_n$- mit n = 0 bis 5,
- $R^2$ ein Wasserstoffatom, wenn X eine Carbamoylgruppe ist, eine Alkylgruppe mit 1 bis 6 Kohlenstoffatomen, eine etherartig gebundene Gruppe der Formel $R^3$-O-$CH_2$-$(CHR^4)_m$-$CH_2$-, in der
- $R^3$ ein Wasserstoffatom oder eine Alkylgruppe mit 1 bis 6 Kohlenstoffatomen,
- $R^4$ eine Hydroxygruppe oder eine Alkyloxygruppe mit 1 bis 3 Kohlenstoffatomen und m = O bis 2 sind, und
- $R^2$ weiter eine etherartig gebundene Gruppe der Formel H-$(CH_2)_a$-(O-$(CH_2)_b)_c$- mit a = 0 bis 4, b =1 bis 4 und c = 1 bis 7 oder einen O-glycosidisch gebundenen Rest der Formel

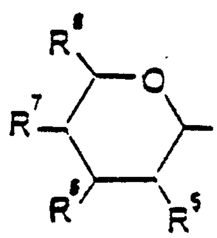

in dem

- $R^5$, $R^6$ und $R^7$ unabhängig voneinander ein Wasserstoffatom oder eine Hydroxygruppe und
- $R^8$ ein Wasserstoffatom, eine Methyl- oder Hydroxymethylgruppe sind,
- X eine Methylen- oder eine Carbamoylgruppe oder eine kovalente Bindung zwischen $R^1$ und dem C-2-Atom,
- $A^1$ und $A^2$ gleich sind und die Hydroxygruppe, Chlorid, Bromid, Jodid, Nitrat, Acetat oder Trifluoracetat oder
- $A^1$ Sulfat oder Carbonat und $A^2$ $H_2O$ oder
- $A^1$ und $A^2$ zusammen das Dianion einer organischen Säure wie Oxal-, Malon-, Hydroxymalon-, Ethylmalon-, 1,1-oder 1,2-Cyclobutan-dicarbon-, Phthal-, 3- oder 4-Carboxyphthal-, 3,4-Dicarboxyphthal oder N-(Carbamoylmethyl)-iminodiessigsäure darstellen,

dadurch gekennzeichnet, daß man eine Diamino-Verbindung der Formel II

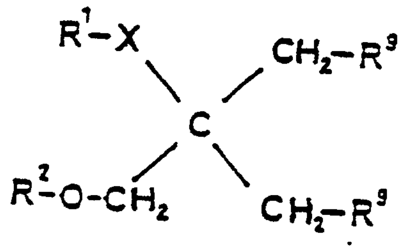

II

worin

$R^1$, $R^2$ und X die Bedeutung nach Anspruch 1 haben und

$R^9$ eine Azido oder eine Aminogruppe, die ggf. als Ammoniumsalz vorliegt, bedeuten,

in an sich bekannter Weise mit $K_2PtCl_4$ zu einem Platinkomplex der Formel I umsetzt, aus dem anschließend weitere Derivate der Formel I hergestellt werden.

**Claims**

**Claims for the following Contracting States : AT, BE, CH, DE, FR, GB, IT, LI, LU, NL, SE**

**1.** A compound of the formula I

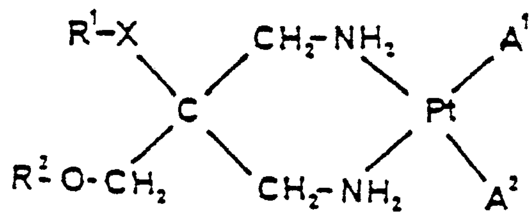

in which

$R^1$ represents a hydrogen atom or an alkyl group of the formula $CH_3(CH_2)_n$- where n = 0 to 5,

$R^2$ represents a hydrogen atom when X is a carbamoyl group, an alkyl group having 1 to 6 carbon atoms, a group, bonded in an ether-like fashion, of the formula $R^3$-O-$CH_2$-$(CHR^4)_m$-$CH_2$- in which

$R^3$ is a hydrogen atom or an alkyl group having 1 to 6 carbon atoms,

$R^4$ is a hydroxyl group or an alkyloxy group having 1 to 3 carbon atoms, and m is 0 to 2, and

$R^2$ further represents a group, bonded in an ether-like fashion, of the formula H-$(CH_2)_a$-(O-$(CH_2)_b)_c$- where a = 0 to 4, b = 1 to 4 and c = 1 to 7, or represents a radical, bonded in a O-glycoside fashion, of the formula

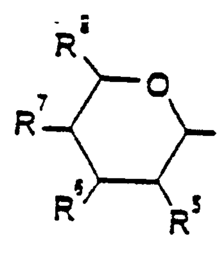

in which

$R^5$, $R^6$ and $R^7$, independently of one another, are a hydrogen atom or a hydroxyl group, and

$R^8$ is a hydrogen atom, a methyl group or a hydroxy methyl group,

X represents a methylene group or a carbamoyl group or a covalent bond between $R^1$ and the 2-carbon atom,

$A^1$ and $A^2$ are identical and represent the hydroxyl group, chloride, bromide, iodide, nitrate, acetate or trifluoroacetate, or

$A^1$ represents sulfate or carbonate and $A^2$ represents $H_2O$ or

| | |
|---|---|
| $A^1$ and $A^2$ | together represent the dianion of an organic acid such as oxalic, malonic, hydroxymalonic, ethylmalonic, 1,1- or 1,2-cyclobutanedicarboxylic, phthalic, 3- or 4-carboxyphthalic, 3,4-dicarboxyphthalic or N-(carbamoylmethyl)-iminodiacetic acid. |

**2.** A compound of the formula II, which is used as a precursor for the preparation of a compound as claimed in claim 1,

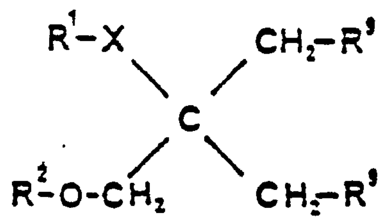

II

in which

| | |
|---|---|
| $R^1$ | is a hydrogen atom or an alkyl group of the formula $CH_3(CH_2)_n$ where n = 0 to 5, |
| $R^2$ | is a hydrogen atom, |
| $R^9$ | is an azido group, and |
| X | is a methylene group or a carbamoyl group or a covalent bond between $R^1$ and the 2-carbon atom. |

**3.** A compound of the formula II, which is used as an intermediate for the preparation of a compound as claimed in claim 1,

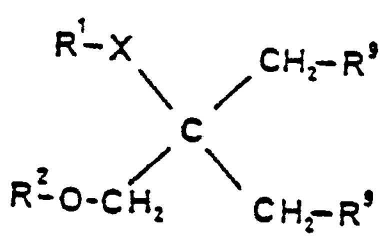

II

in which

| | |
|---|---|
| $R^1$, $R^2$ and X | have the meaning given in claim 1, and |
| $R^9$ | denotes an azido group or an amino group, which exists, if appropriate, as an ammonium salt. |

**4.** A process for the preparation of a compound as claimed in claim 1, wherein a diamino compound of the formula II as claimed in claim 3 is reacted, in a fashion which is known per se, with $K_2PtCl_4$ to give a platinum complex of the formula I, from which further derivatives of the formula I are subsequently prepared.

**5.** The use of a compound as claimed in claim 1 for the preparation of a medicament.

**Claims for the following Contracting States : ES, GR**

**1.** A process for the preparation of a compound of the formula I

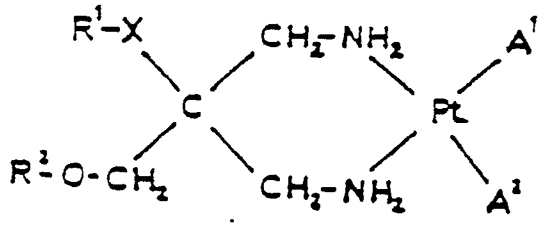

I

in which

$R^1$ represents a hydrogen atom or an alkyl group of the formula $CH_3(CH_2)_n$- where n = 0 to 5,

$R^2$ represents a hydrogen atom when X is a carbamoyl group, an alkyl group having 1 to 6 carbon atoms, a group, bonded in an ether-like fashion, of the formula $R^3$-O-$CH_2$-$(CHR^4)_m$-$CH_2$- in which

$R^3$ is a hydrogen atom or an alkyl group having 1 to 6 carbon atoms,

$R^4$ is a hydroxyl group or an alkyloxy group having 1 to 3 carbon atoms, and m is 0 to 2, and

$R^2$ further represents a group, bonded in an ether-like fashion, of the formula H-$(CH_2)_a$-(O-$(CH_2)_b)_c$- where a = 0 to 4, b = 1 to 4 and c = 1 to 7, or represents a radical, bonded in a O-glycoside fashion, of the formula

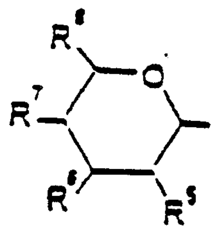

in which

$R^5$, $R^6$ and $R^7$, independently of one another, are a hydrogen atom or a hydroxyl group, and

$R^8$ is a hydrogen atom, a methyl group or a hydroxy methyl group,

X represents a methylene group or a carbamoyl group or a covalent bond between $R^1$ and the 2-carbon atom,

$A^1$ and $A^2$ are identical and represent the hydroxyl group, chloride, bromide, iodide, nitrate, acetate or trifluoroacetate, or

$A^1$ represents sulfate or carbonate and $A^2$ represents $H_2O$ or

$A^1$ and $A^2$ together represent the dianion of an organic acid such as oxalic, malonic, hydroxymalonic, ethylmalonic, 1,1- or 1,2-cyclobutanedicarboxylic, phthalic, 3- or 4-carboxyphthalic, 3,4-dicarboxyphthalic or N-(carbamoylmethyl)-iminodiacetic acid, which comprises reacting a diamino compound of the formula II

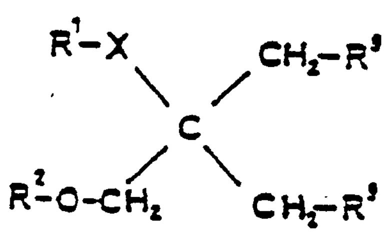

II

in which

$R^1$, $R^2$ and X have the meaning as claimed in claim 1 and

$R^9$ is an azido group or an amino group, which exists, if appropriate, as an ammonium salt, in a fashion which is known per se, with $K_2PtCl_4$ to give a platinum complex of the formula I, from which further derivatives of the formula I are subsequently prepared.

**Revendications**

**Revendications pour les Etats contractants suivants : AT, BE, CH, DE, FR, GB, IT, LI, LU, NL, SE**

1. Composé de formule générale I

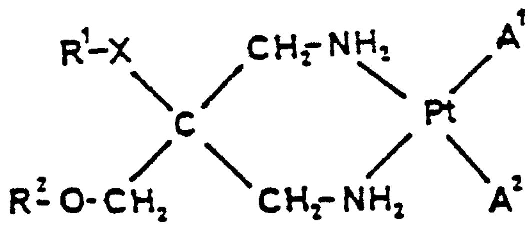

dans laquelle

$R^1$ représente un atome d'hydrogène ou un groupe alkyle de formule $CH_3(CH_2)_n$- avec n = 0 à 5,

$R^2$ représente un atome d'hydrogène lorsque X est un groupe carbamoyle, un groupe alkyle ayant de 1 à 6 atomes de carbone, un groupe de formule $R^3$-O-$CH_2$-$(CHR^4)_m$-$CH_2$-, lié par une liaison éther, dans lequel

$R^3$ représente un atome d'hydrogène ou un groupe alkyle ayant de 1 à 6 atomes de carbone,

$R^4$ représente le groupe hydroxy ou un groupe alkyloxy ayant de 1 à 3 atomes de carbone, et m va de 0 à 2, et

$R^2$ représente en outre un groupe de formule H-$(CH_2)_a$-$(O(CH_2)_b)_c$- avec a = 0 à 4, b = 1 à 4 et c = 1 à 7, ou un radical, lié par une liaison O-glucosidique, de formule

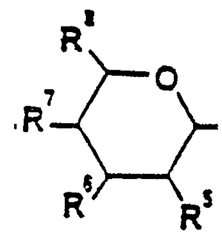

dans laquelle

| | |
|---|---|
| $R^5$, $R^6$ et $R^7$ | sont, indépendamment les uns des autres, un atome d'hydrogène ou le groupe hydroxy, et |
| $R^8$ | est un atome d'hydrogène ou le groupe méthyle ou hydroxyméthyle, |
| X | représente le groupe méthylène ou carbamoyle ou une liaison covalente entre $R^1$ et l'atome de carbone 2. |
| $A^1$ et $A^2$ | sont identiques et représentent le groupe hydroxy ou l'ion chlorure, bromure, iodure, nitrate, acétate ou trifluoroacétate, ou |
| $A^1$ | représente l'ion sulfate ou carbonate et $A^2$ représente $H_2O$ ou |
| $A^1$ et $A^2$ | représentent ensemble le di-anion d'un acide organique, tel que l'acide oxalique, malonique, hydroxymalonique, éthylmalonique, 1,1- ou 1 ,2-cyclobutanedicarboxylique, phtalique, 3- ou 4-carboxyphtalique, 3,4-dicarboxyphtalique ou N-(carbamoylméthyl)-iminodiacétique. |

2. Composé de formule générale **II**, qui est utilisé en tant que précurseur pour la préparation du composé selon la formule **I**,

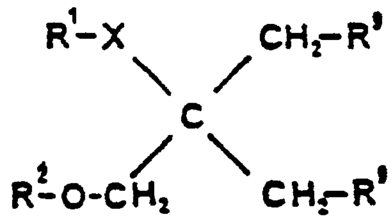

II

dans laquelle

$R^1$ est un atome d'hydrogène ou un groupe alkyle de formule $CH_3(CH_2)_n$ avec n = 0 à 5,

$R^2$ est un atome d'hydrogène,

$R^9$ est un groupe azido et

X est le groupe méthylène ou carbamoyle, ou une liaison covalente entre $R^1$ et l'atome de carbone 2.

**3.** Composé de formule générale II qui est utilisé en tant que produit intermédiaire pour la préparation des composés selon la revendication 1,

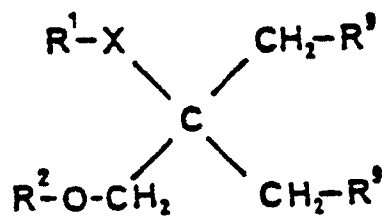

II

dans laquelle

$R^1$ , $R^2$ et X ont les significations données dans la revendication 1 et

$R^9$ représente un groupe azido ou un groupe amino qui se trouve éventuellement sous forme de sel d'ammonium.

**4.** Procédé pour la préparation d'un composé selon la revendication 1 caractérisé en ce que l'on fait réagir un composé diaminé de formule II selon la revendication 3, d'une façon connue en soi, avec $K_2PtCl_4$, pour aboutir à un complexe de platine, à partir duquel on prépare ensuite d'autres dérivés de formule I.

**5.** Utilisation d'un composé selon la revendication 1, pour la fabrication d'un médicament.

**Revendications pour les Etats contractants suivants : ES, GR**

**1.** Procédé pour la préparation d'un composé de formule générale I

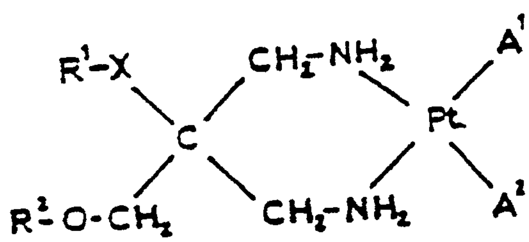

I

dans laquelle

$R^1$ représente un atome d'hydrogène ou un groupe alkyle de formule $CH_3(CH_2)_n$- avec n = 0 à 5,

$R^2$ représente un atome d'hydrogène lorsque X est un groupe carbamoyle, un groupe alkyle ayant de 1 à 6 atomes de carbone, un groupe de formule $R^3$-O-$CH_2$-$(CHR^4)_m$-$CH_2$-, lié par une liaison éther, dans lequel

$R^3$ représente un atome d'hydrogène ou un groupe alkyle ayant de 1 à 6 atomes de carbone,

$R^4$ représente le groupe hydroxy ou un groupe alkyloxy ayant de 1 à 3 atomes de carbone, et m va de 0 à 2, et

$R^2$ représente en outre un groupe de formule $H-(CH_2)_a-(O(CH_2)_b)_c-$ avec a = 0 à 4, b = 1 à 4 et c = 1 à 7, ou un radical, lié par une liaison O-glucosidique, de formule

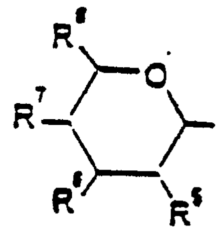

dans laquelle

$R^5$, $R^6$ et $R^7$ sont indépendamment les uns des autres un atome d'hydrogène ou le groupe hydroxy, et

$R^8$ est un atome d'hydrogène ou le groupe méthyle ou hydroxyméthyle,

X représente le groupe méthylène ou carbamoyle ou une liaison covalente entre $R^1$ et l'atome de carbone 2,

$A^1$ et $A^2$ sont identiques et représentent le groupe hydroxy ou l'ion chlorure, bromure, iodure, nitrate, acétate ou trifluoroacétate, ou

$A^1$ représente l'ion sulfate ou carbonate et $A^2$ représente $H_2O$ ou

$A^1$ et $A^2$ représentent ensemble le di-anion d'un acide organique, tel que l'acide oxalique, malonique, hydroxymalonique, éthylmalonique, 1,1- ou 1,2-cyclobutanedicarboxylique, phtalique, 3- ou 4-carboxyphtalique, 3,4-dicarboxyphtalique ou N-(carbamoylméthyl)-iminodiacétique

caractérisé en ce que l'on fait réagir un composé diaminé de formule II

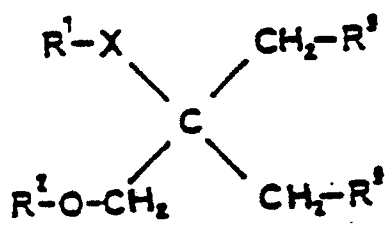

II

dans laquelle

$R^1$ $R^2$ et X ont les significations données plus haut et

$R^9$ représente un groupe azido ou un groupe amino qui se trouve éventuellement sous forme de sel d'amminium,

d'une façon connue en soi avec $K_2PtCl_4$, pour aboutir à un complexe de platine de formule I, à partir duquel on prépare ensuite d'autres dérivés de formule I.